(19) Europäisches Patentamt
European Patent Office
Office européen des brevets

(11) EP 1 666 031 A1

(12) **EUROPEAN PATENT APPLICATION**
published in accordance with Art. 158(3) EPC

(43) Date of publication:
**07.06.2006 Bulletin 2006/23**

(21) Application number: **04772938.9**

(22) Date of filing: **06.09.2004**

(51) Int Cl.:
***A61K 9/51*** *(1990.01)* ***A61K 47/32*** *(1990.01)*
***A61K 47/34*** *(1990.01)* ***A61K 47/36*** *(1990.01)*
***A61K 47/42*** *(1990.01)* ***A61P 5/10*** *(2000.01)*
***A61P 5/14*** *(2000.01)* ***A61P 5/24*** *(2000.01)*
***A61P 25/04*** *(2000.01)* ***A61P 35/00*** *(2000.01)*

(86) International application number:
**PCT/JP2004/013256**

(87) International publication number:
**WO 2005/023230 (17.03.2005 Gazette 2005/11)**

(84) Designated Contracting States:
**AT BE BG CH CY CZ DE DK EE ES FI FR GB GR HU IE IT LI LU MC NL PL PT RO SE SI SK TR**

(30) Priority: **04.09.2003 JP 2003312848**

(71) Applicant: **NanoCarrier Co., Ltd.**
**Kashiwa-shi,**
**Chiba-ken 277-0882 (JP)**

(72) Inventors:
- **OGAWA, Yasuaki**
**Otokuni-gun,**
**Kyoto 618-0071 (JP)**
- **NAGASAKI, Shoko**
**Moriya-shi, Ibaraki 302-0128 (JP)**
- **TSUCHIYA, Chieko**
**Tokyo 177-0043 (JP)**
- **HIGASHI, Sohei**
**Nakano-ku, Tokyo 164-0003 (JP)**
- **SAITO, Hiroyuki**
**Matsudo-shi,**
**Chiba 271-0086 (JP)**

(74) Representative: **Albrecht, Thomas**
**Kraus & Weisert**
**Patent- und Rechtsanwälte**
**Thomas-Wimmer-Ring 15**
**80539 München (DE)**

(54) **COMPOSITION CONTAINING NANOPARTICLES CONTAINING WATER-SOLUBLE BASIC DRUG ENCAPSULATED THEREIN**

(57) A drug and a biodegradable polymer having at least one carboxyl group are encapsulated into nanoparticle which is formed by a block copolymer having a hydrophilic segment and a hydrophobic segment. This invention thus provides a drug-encapsulated nanoparticle which shows an increased *in vivo* drug-stability.

EP 1 666 031 A1

## Description

Technical Field

This invention relates to a composition which contains stable nanoparticles for medicinal use, each of which contains water-soluble, basic low-molecular compound encapsulated therein.

Background Arts

A lot of techniques have been disclosed with regard to drug-containing nanoparticles (or nanocapsules). For instance, Yokoyama et al. proposed a polymer micelle as nanoparticles which encapsulate therein a drug slightly soluble in water, with use of a block copolymer which comprises a hydrophilic polymer and a hydrophobic polymer (see, for example, JP-B-2777530 or its corresponding US-A-5,449,513). According to these patent documents, it is compounds slightly soluble in water that can be encapsulated in polymer micelle stably. As to how to encapsulate adriamycin, a water-soluble compound, in polymer micelle, an idea has been proposed according to which the drug is chemically bound to the side chain of hydrophobic polymer and is thereby encapsulated in polymer micelle (see, for example, JP-B-2517760 or its corresponding US-A-5,412,072). As an example of polymer micelle in which to efficiently encapsulate an electrically charged drug, e.g., positively-charged basic peptide, there has been disclosed a method wherein a negatively-charged acidic group is introduced to the side chain of hydrophobic polymer, so that an electrostatic interaction may be caused between positive charge and negative charge, and the drug is thereby encapsulated in polymer micelle (see, for example, JP-B-2690276 or EP-A-0 721 776). The methods as disclosed in these patent documents make it possible to efficiently encapsulate water-soluble drug in polymer micelle, and thus produced nanoparticles (polymer micelle) exist stably in aqueous solution. In the presence of salt, however, drugs may be rapidly released from polymer micelle according to circumstances. Although liposome can be mentioned as nanoparticles in which to encapsulate water-soluble drugs (see Pharm Tech Japan, 19 (2003), 99-110), drug-encapsulation rate is low, the stability of the drugs in living body is insufficient, and industrial production is difficult, which problems have yet to be solved.

Disclosure of Invention

As stated above, although it was possible to stably and efficiently encapsulate water-soluble and basic (positively charged) drugs in nanoparticles such as polymer micelle, it was hard to encapsulate them stably in a solution wherein salt existed. The inventors of the present invention actually encapsulated water-soluble, low-molecular drugs in nanoparticles (polymer micelle) in accordance with the method as defined in JP-B-2690276 (corresponding to EP-A-0 721 776), and evaluated stability. Although the nanoparticles were stable in an aqueous solution, the drugs were readily released from capsules when put in an environment which was abundant in electrostatic counter ions. This means that basic drugs are easily released from nanoparticles in an environment which is rich in counter ions such as the interior of vein of living body, and that the expected effects are hardly given. In order that nanoparticles may show their function, and that water-soluble drugs may exhibit their efficacy effectively in a living body, it is essentially required that nanoparticles which contain drugs encapsulated therein should keep stable for a considerably long time without releasing the drugs even in an environment (in particular, in a living body) which is rich in counter ions. The purpose of this invention is to provide a nanoparticle composition which satisfies such a need.

With a view to solving the above-mentioned problems, the inventors of this invention studied various means by which to encapsulate water-soluble and basic drugs in nanoparticles. As a result, they have found quite unexpectedly that, when fine particles are formed with said drugs using a block copolymer having a hydrophilic segment and a hydrophobic segment, together with a biodegradable polymer having at least one carboxyl group in a molecule, said drugs are not only effectively encapsulated in nanoparticles, but also kept stable in said nanoparticles even in a living body.

Thus, the above-mentioned problems are solved by a composition containing drug-encapsulated nanoparticles each of which comprises three components of a) water-soluble and basic drug, b) a biodegradable polymer having at least one carboxyl group in a molecule, and c) a block copolymer having a hydrophilic segment and a hydrophobic segment.

This invention provides nanoparticles which contain water-soluble and basic drug effectively encapsulated therein, and the encapsulated drug can be kept stable in a physiological environment. The following are concrete explanations on this invention.

Water-soluble and basic drug in this invention means a drug which has a solubility of at least 0.1 mg/mL in water at room temperature, preferably at least 0.5 mg/mL in water, more desirably 1 mg/mL in water. Drugs which have a solubility of less than 0.1 mg/mL in water also fall under the scope of this invention so long as they are basic, positively charged in an aqueous medium and show the effects of this invention. Any kind of compound is usable as drug for this invention if only it has some physiologically useful action on living body when administered therein. Examples of such

a drug include, although not restrictive, basic polypeptides such as thyrotropin-releasing hormone (TRH), gonadotropic hormone-releasing hormone (GnRH), enkephalin, growth hormone-releasing hormone (GHRP), and their homologues or analogues.

Said analogue means a polypeptide which has the activity of one of hormones as recited above, and in which at least one amino acid residue is deleted, substituted or added. Such a polypeptide may be one which is classified in so-called oligopeptide so long as it shows the desired activity. Hence, in this specification, the prefix "poly" includes "oligo" where appropriate. This applies also to block copolymer. In this invention, the molecular weight of polypeptide is at most 5000.

Examples of another type of drug include, although not restrictive, amino glycoside such as gentamicin, water-soluble camptothecin derivative such as topotecan, and the like. Publicly known concrete examples of said derivative are those which are mentioned in US-A-4,473,692, US-A-4,545,880, EP-A-321122, JP-A-5222048, JP-T-8509740 (corresponding to WO 94/25466), JP-T-850221 (corresponding to WO 94/11377), and also in JP-A-4139187, JP-A-4139188, JP-A-5279370, JP-T-8505626, JP-T-8509244, JP-T-10503525, JP-A-11140085, JP-T-2001506270, JP-T-2001506282, and the like, and which show water-solubility.

More specifically, there can be mentioned a derivative having a side chain which carries amino group, mono- or disubstituted amino group (which includes a case where the two substituents are taken together to form a ring with the nitrogen atom to which they are bound) at one or more positions selected from 5-, 7-, 9-, 10- and 11-positions of camptothecin structure as follows:

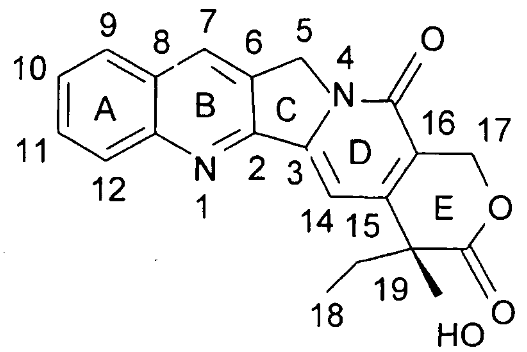

Typical examples of the above-mentioned side chain have a formula as follows:

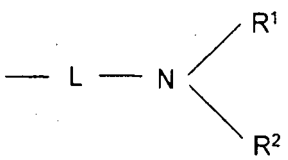

such that the molecule as a whole is more desirably water-soluble.

In the above formula, L denotes a divalent group which connects, at the above-mentioned position, camptothecin structure and amino group, e.g., $C_{1-4}$ alkylene, ester bond (-OCO-), carbonyl (-CO-) or iminocarbonyl (=C=NH) or the like, or a single bond; $R^1$ and $R^2$ either independently denote hydrogen atom, $C_{1-4}$ alkyl, $C_{3-7}$ cycloalkyl, $C_{3-7}$ cycloalkyl $C_{1-4}$ alkyl, $C_{3-6}$ alkenyl, hydroxyl $C_{1-6}$ alkyl or $C_{1-4}$ alkoxy $C_{1-4}$ alkyl, or, taken together, may form, together with the nitrogen atom to which they are bound, a saturated 5- to 8-membered carbon ring or heterocycle which may contain one oxygen, nitrogen or sulfur atom as a member, said carbon ring or heterocycle being, according to circumstances, substituted by one or more the same or different substituents selected from the group consisting of $C_{1-4}$alkyl, halogen, amino, hydroxyl, piperidino and piperazino.

The other positions which have no such side chain as mentioned above may be substituted by $C_{1-4}$ alkyl, $C_{1-4}$ hydroxyl alkyl, hydroxyl, alkylene dioxy ($-O(CH_2)_m-O-$; m denotes an integer of 1 or 2), halogen (fluorine, chlorine or bromine), or the like.

Camptothecin derivatives (there exist a lot of camptothecin derivatives whose anti-tumor activity is equivalent to, or higher than, that of camptothecin) having such preferable substituents as mentioned above are, when used in the form of a substantially free base, stably encapsulated in nanoparticles of this invention at a high content (although not

restrictively, at least about 0.5 % by weight, preferably at least about 2 % by weight, or, according to circumstances, more than 15 % by weight, based on the total weight of drug-containing nanoparticles). The above term "substantially" means that camptothecin derivatives in the form of acid addition salt account for at most 5 % by weight, preferably 0 % by weight. Moreover, even though these camptothecin derivative are slightly soluble in water, thus prepared drug-loaded polymer micelle strongly solubilizes said camptothecin derivative to be apparently water-soluble. The term "apparently" includes the state where nanoparticles are dispersed, and they are seemingly completely dissolved. This invention provides stable and high content drug-loaded nanoparticles as mentioned above even when the above-mentioned camptothecin derivatives are in the form of a free base and water-soluble (in this invention, "water-soluble" means that at least 0.5 mg of drug is dissolved in 1 mL of water at 25°C).
Details will be given later.

Examples of in particular preferably usable camptothecin derivatives include, although not restrictive, 9-N,N-dimethylethyl-10-hydroxycamptothecin (topotecan), N-desmethyl-topotecan, 7-ethyl-10-[1-(4-piperidino)piperidino]carbonyloxy camptothecin, 7-ethyl-9-(N-methyl-N-phenyl) amidino camptothecin, etc.

Examples of biodegradable polymer having at least one carboxyl group in a molecule in this invention include those which have one carboxylic group at a terminal of molecule, such as poly(lactic acid), poly(lactic-coglycolic acid) [in said copolymer, units originated in lactic acid and glycolic acid may exist in a block-like manner or at random], poly (butyric acid), poly(caprolactone), and the like, and also those which have introduced therein another carboxylic group via hydroxyl group on the other terminal of poly(lactic acid) or poly(lactic-coglycolic acid) to be a half-ester derivative with polycarboxylic acid (e.g., citric acid, maleic acid, succinic acid, itaconic acid, etc.), which are not restrictive so long as the effects of this invention are given. These biodegradable polymers may be used singly or in combination of two or more species. The polymers which are mentioned above as typical can be prepared, for instance by the ring-opening polymerization of one or more corresponding cyclic monomers (e.g., lactide, glycolide and lactones) or by the polycondensation of corresponding non-cyclic monomers. The weight average molecular weight of these polymers is, although not restrictive, preferably at most 30,000, more desirably in a range of 3,000 to 30,000.

It is considered that, according to this invention, the above-mentioned water-soluble, basic drug and the above-mentioned polymer form a composite by interaction between positively charged moiety and carboxyl group, and are thereby efficiently and stably encapsulated in the hydrophobic core of polymer micelle comprising block copolymer as mentioned later, although this invention is not restricted by such a theory. For instance, an example has been found out that even a water-soluble basic drug which is insoluble in dichloromethane can be dissolved in dichloromethane when made co-existent with poly(lactic acid) or poly(lactic-coglycolic acid). This fact suggests that a biodegradable polymer having a carboxyl group in a molecule and a water-soluble basic drug are dissolved owing to interaction between themselves. Thus, in this invention, the proportion of biodegradable polymer to water-soluble basic drug is, in a molar ratio (i.e., the ratio of the average number of molecules of polymer to the number of molecules of drug), preferably in a range of 2 to 0.1, more desirably in a range of 1.5 to 0.5, most desirably 1, although not restrictive.

Block copolymer in this invention comprises hydrophilic polymer segment and hydrophobic polymer segment, and forms polymer micelle (so-called core-shell type nanoparticle wherein hydrophobic polymer segment mainly constitutes the core, and hydrophilic polymer segment mainly constitutes the shell) in the presence of water. The segments may be of any kind so long as they exhibit the effects of this invention. Concrete examples of hydrophilic segment include those selected from the group consisting of poly(ethyleneoxide), poly(vinylalcohol), poly(vinylpyrrolidone), poly(N,N-dimethylacrylamide) and dextran. Among these, when poly(ethyleneoxide) is contained as a hydrophilic segment, nanoparticle can have a structure wherein its surface is covered with polyethylene glycol (PEG). Thus, when intravenously administered in a living body, said nanoparticles can favorably exert a function to avoid being captured by reticuloendothelial system (RES). Examples of hydrophobic segment include those selected from the group consisting of poly(β-alkylaspartate), poly(β-alkylaspartatecoaspartic acid), poly(β-aralkylaspartate), poly(β-aralkylaspartatecoaspartic acid), poly(γ-alkylglutamate), poly(y-alkylglutamatecoglutamic acid), poly(γ-aralkylglutamate), poly(β-alkylaspartamide), poly (β-alkylaspartamide-coaspartic acid), poly(β-aralkylaspartamide), poly(β-aralkylaspartamide-coaspartic acid), poly(γ-alkylglutamide), poly(γ-alkylglutamide-coglutamic acid), poly(γ-aralkylglutamide), poly(γ-aralkylglutamide-coglutamic acid), poly(lactide), poly(lactidecoglycolide), poly(ε-caprolactone), poly(δ-valerolactone) and poly(γ-butyrolactone).

A concrete example of preferable block copolymer which comprises hydrophilic polymer segment and hydrophobic polymer segment has formula (I) or (II) as follows:

$$R_1-(OCH_2CH_2)_n-L_1-((\underset{CH_2COOH}{\underset{|}{COCHNH}})_x \cdot (\underset{CH_2COO-R_5}{\underset{|}{COCHNH}})_y)-R_2 \qquad (I)$$

$$R_3-(OCH_2CH_2)_n-L_2-((NH\underset{\displaystyle CH_2COOH}{\underset{|}{C}}HCO)_x\cdot(NH\underset{\displaystyle CH_2COO-R_5}{\underset{|}{C}}HCO)_y)-R_4 \qquad (II)$$

wherein $R_1$ and $R_3$ independently denote a hydrogen atom or a lower alkyl group which is either substituted by a functional group which may be protected or unsubstituted; $R_2$ denotes a hydrogen atom, a saturated or unsaturated $C_1$-$C_{29}$ aliphatic carbonyl group or an aryl carbonyl group; $R_4$ denotes a hydroxyl group, a saturated or unsaturated $C_1$-$C_{30}$ aliphatic oxy group or an aryl-lower alkyloxy group; $R_5$ denotes a benzyl group, alkylbenzyl group or an aryl group; $L_1$ and $L_2$ independently denote a linker; n denotes an integer of 10 to 2500; and x and y are the same or different, and each denote an integer such that the total of x and y is 10 to 300, provided that the proportion of x:y falls preferably in the range of 2-0 : 8-10, or that, more desirably, x is zero (0), and that, when x and y exist, the recurring units of x and y are each present at random. $L_1$ preferably denotes, not restrictively, a group selected from the group consisting of -NH-, -O-, -CO-, -$CH_2$-, -O-Z-S-Z-, -O-Z-NH- and -OCO-Z-NH- (Z independently denotes a $C_1$-$C_4$ alkylene group), and $L_2$ preferably denotes, not restrictively, a group selected from the group consisting of -OCO-Z-CO-, -NHCO-Z-CO- and -O-Z-NH-(Z independently denotes a $C_1$-$C_4$ alkylene group). These block copolymers can be produced by the method as disclosed in JP-B-2777530 or JP-B-2690276, or by a modification thereof.

Examples of block copolymer whose hydrophobic segment is selected from the group consisting of poly(lactide), poly(lactide-co-glycolide), poly(ε-caprolactone), poly(δ-valerolactone) or poly(γ-butyrolactone) include those which are disclosed in WO 96/32434, WO 96/33233 and WO 97/06202, and those which are produced by the methods as disclosed therein or by the modification thereof.

The above-mentioned modification means an appropriate improvement by combining methods which are well known in this field and those which are disclosed in the above-recited patent documents. The above-mentioned groups are also explained in said patent documents. Saturated or unsaturated $C_1$-$C_{29}$ aliphatic carbonyl group and saturated or unsaturated $C_1$-$C_{30}$ aliphatic oxy group mean a group having $C_1$-$C_{29}$ or $C_1$-$C_{30}$ hydrocarbon moiety which may be branched and may have one or more unsaturated bond. Representative examples of said hydrocarbon moiety is alkyl, e.g., lower alkyl such as methyl, ethyl, *n*-propyl, *i*-propyl, *n*-butyl, *t*-butyl, *n*-hexyl; middle alkyl having more carbon atoms; and tetradecyl, hexadecyl, octadecyl and docosanyl. These groups may be substituted by one or more halogen (e.g., fluorine, chlorine and bromine). Middle and higher alkyls may be substituted by one hydroxyl group. The above explanation on alkyl is applied also to alkyl of $C_1$-$C_{12}$ alkyl and $C_1$-$C_{22}$ alkylcarbonyl. Another example of hydrocarbon portion is aralkyl, e.g., phenyl- $C_1$-$C_4$ alkyl such as benzyl, whose benzene ring may be substituted by one to three halogen atoms or by a lower alkyl.

Preferable among the above-mentioned block copolymers are those wherein the side chain of poly(aspartic acid) in formula (I) or (II) is aliphatic ester, aliphatic amide which corresponds to said aliphatic ester, or benzylester. Also preferable are those wherein poly(aspartate) segment in formula (I) or (II) has been substituted with poly(glutamate) segment.

The proportion of block copolymer used in the composition of this invention is not limited so long as nanoparticles are formed. The higher the proportion of block copolymer used is, the smaller particle is likely to be formed. Generally, block copolymer is used in an amount of from half to several times the total weight of biodegradable polymer and water-soluble basic drug. According to the difference of alkyl chain in aliphatic ester, the encapsulating rate of biodegradable polymer and water-soluble basic drug differs. Thus, optimal proportion is chosen for use.

Nanoparticles of this invention generally include those of the size up to several microns. In view of the avoidance of uptake by RES, however, the average particle size is preferably 300 nm or less, more desirably 30 to 200 nm. The stability of nanoparticles is evaluated by various methods, e.g., judgement from the change of drug concentration in blood after administered in animal, judgement from the stability or releasing rate of drug in 50 % plasma (originated in any kind of animal)-containing phosphate buffer solution, or judgement from the releasing ratio of drug in phosphate buffered saline. Said releasing ratio can be found by the following method. Nanoparticles are stirred and dispersed in phosphate buffered saline at 37°C, and are, after a certain time (5 minutes), subjected to ultrafiltration (e.g., with use of ultrafiltration membrane whose MWCO is 100,000), and, then, the amount of drug in filtrate is measured. From thus obtained results, the ratio of releasing of drug from the nanoparticles is calculated.

Nanoparticles are produced by various methods. The following is a typical one. Water-soluble basic drug and biodegradable polymer having a carboxyl group are dissolved or dispersed in a suitable organic solvent. Examples of representative organic solvent used include acetone, dichloromethane, dimethylformamide, dimethyl sulfoxide, acetonitrile, tetrahydrofuran and methanol. These solvents may also be used in combination. If necessary, a small amount of water may be mixed. Then, block copolymer is added, and dissolved or dispersed. After the block copolymer has been sufficiently dissolved or dispersed, said organic solvent is removed by evaporation. To paste or solid matter which

is obtained after the solvent has been removed, water or an aqueous solution containing suitable additive such as stabilizer is gradually added at low temperature, and the resultant mixture is vigorously stirred so that said paste or solid matter may be gradually dispersed or dissolved in water. The resultant dispersion or solution is uniformly dispersed and atomized by sonification or the like, and, thus, nanoparticles are obtained. Otherwise, block copolymer may be added simultaneously with drug. In another method, block copolymer is previously dispersed or dissolved in water, and, then, the resultant aqueous solution or dispersion is added to the above-mentioned paste or solid matter, and the resultant mixture is vigorously stirred. This method also gives nanoparticles.

In the following, this invention is concretely explained by working examples.

Example 1

Polyethylene glycol (molecular weight: 12000) - co-poly(benzyl-L-aspartate) (degree of polymerization of aspartic acid: 50) (esterification rate: 100 %) (hereinafter referred to as PEG-PBLA 12-50) was used as a block copolymer. 50 mg of PEG-PBLA 12-50, 1 mg of topotecan and 20 mg of PLA-20000 were put into a 9 mL screwed tube bottle, and were then dissolved in 2 mL of dichloromethane. The resultant solution was then dried and solidified with nitrogen blowing, and a film-like matter was obtained, which was subsequently further dried under reduced pressure for about 30 minutes to one hour. To the film-like matter, 3 mL of water was added, and the resultant mixture was stirred for a whole day and night at 4°C. The mixture was thereafter subjected to sonification for five minutes, and, then, large particles and extraneous matters were filtered out with a membrane having a pore size of 0.8 μm, and, thus, topotecan-encapsulated nanocapsules were prepared. Furthermore, unencapsulated drug was filtered out by Amicon Ultra, an ultrafiltration membrane (MWCO: 100,000).

Encapsulating rate was measured using an ultrafiltration membrane (Microcon YM-100; MWCO: 100,000). One hundred μL of sample from which unencapsulated drug had been removed by the above-mentioned operation was set in Microcon YM-100, and was then centrifuged for five minutes at 4°C, 10000 rpm, to give a filtrate. The amount of topotecan in the sample (A) and the amount of topotecan in the filtrate (B) were measured with HPLC, and, thus, drug-encapsulated rate was calculated according to the following formula. As a result, it was found that 91.1 % of topotecan had been encapsulated in particles.

$$\text{Encapsulating rate (\%)} = \frac{(A - B) \times 100}{A}$$

Particle size was measured with dynamic light scattering photometer (DLS). It was found that average particle size was about 130 nm. The stability of topotecan-encapsulating nanoparticles was evaluated in the following manner.

Topotecan (TPT)-encapsulating rate in nanoparticles when PBS buffer solution had been added was measured, and, thus, the rate of releasing of TPT caused by the addition of salt was determined. To 90 μL of sample from which unencapsulated drug had been removed by ultrafiltration membrane in the above-mentioned manner, 10 μL of PBS buffer solution was added, and the resultant mixture was stirred by vortex for 30 seconds. As soon as stirring was over, encapsulating rate was measured in the same manner as mentioned above, and, thus, the proportion of TPT remaining in the particles was confirmed. As a result, it was found that 61.6 % of topotecan were encapsulated or incorporated in nanoparticles.

Fifty μL of TPT-containing PLA particle formulation or 50 μL of aqueous TPT solution (0.3 mg/mL) containing 50 mg/mL of PEG-4000 and 50 mg/mL of mannitol was freeze-dried. This freeze-dried product (control) and the above-mentioned nanoparticles were each dissolved in 1 mL of 50 % human plasma (diluted with PBS), and were incubated at 37°C. For the purpose of investigating the ratio of ring-opening of lactone ring of TPT with lapse of time, 100 μL was taken out from the incubated sample after 0 hour, 2 hours and 4 hours, and was added to 900 μL of methanol. Plasma protein was denatured while the equilibrium of TPT structure was maintained, and, then, the samples were centrifuged at 10000 rpm for 10 minutes so that protein components were precipitated, and, then, the concentration of lactone ring-opened TPT and that of lactone ring-closed TPT in supernatant were measured with HPLC. Results are shown in Table 1 below. An aqueous solution of TPT (pH 3; phosphate hydrochloride buffer) was used for control.

Table 1. Change with time of the ratio of ring-closed TPT in 50 % human plasma

| Item | 0 Hour | 2 Hours | 4 Hours |
|---|---|---|---|
| Control | 97.5 % | 24.6 % | 19.4 % |

Table continued

| Item | 0 Hour | 2 Hours | 4 Hours |
|---|---|---|---|
| Nanoparticles | 96.3 % | 51.6 % | 46.4 % |

Example 2

(Use of a different lot block copolymer from Example 1)

Polyethylene glycol (molecular weight: 12000) - co-poly(benzyl-L-aspartate) (degree of polymerization of aspartic acid: 50) (esterification rate: 100 %) (hereinafter referred to as PEG-PBLA 12-50) was used as block copolymer. 50 mg of PEG-PBLA 12-50, 1 mg of TPT and 20 mg of PLA-20000 were put into a 9 mL screwed tube bottle, and were then dissolved in 2 mL of dichloromethane. The resultant solution was then dried and solidified with nitrogen blowing, and a film-like matter was obtained. To the film-like matter, 3 mL of water was added, and the resultant mixture was stirred vigorously for a whole day and night at 4°C. The mixture was thereafter subjected to sonification for five minutes, and, then, large particles and extraneous matters were filtered out with a membrane having a pore size of 0.8 μm, and, thus, TPT-containing micellar nanoparticles were prepared. Furthermore, unencapsulated drug was removed by Amicon Ultra, an ultrafiltration membrane (MWCO: 100,000).

Encapsulating rate was measured with an ultrafiltration membrane (Microcon YM-100; MWCO: 100,000). One hundred μL of sample from which unencapsulated drug had been removed by the above-mentioned operation was set in Microcon, and was then centrifuged for five minutes at 4°C, 10000 rpm, to give a filtrate. The amount of TPT in the sample immediately after prepared (A) and the amount of TPT in the filtrate (B) were measured with HPLC, and, thus, drug-encapsulating rate was calculated according to the following formula. As a result, it was found that 91.4 % of topotecan had been encapsulated in nanoparticles.

$$\text{Encapsulating rate (\%)} = \frac{(A - B) \times 100}{A}$$

Particle size was measured with DLS. It was found that average particle size was about 130 nm. The stability of TPT-containing nanoparticles was evaluated in the following manner.

Topotecan (TPT)-encapsulating rate in nanoparticles when PBS buffer solution had been added was measured, and, thus, the rate of releasing of TPT caused by the addition of salt was determined. To 90 μL of samples from which unencapsulated drug had been removed by ultrafiltration membrane in the above-mentioned manner, 10 μL of PBS buffer solution was added. Then, encapsulating rate was measured in the same manner as mentioned above, and, thus, the ratio of TPT remaining in the particles was confirmed. As a result, it was found that 40.0 % of TPT had remained in nanoparticles.

Fifty μL of TPT-containing nanoparticle formulation or a 50 μL of aqueous TPT solution (0.3 mg/mL) containing 50 mg/mL of PEG-4000 and 50 mg/mL of mannitol was freeze-dried, and was dissolved in 1 mL of 50 % human plasma (diluted with PBS), and were incubated at 37°C. For the purpose of investigating the ratio of ring-opening and ring-closing of lactone ring of TPT with lapse of time, 100 μL of incubated sample was taken out after 0 hour, 2 hours and 4 hours, and was added to 900 μL of methanol. Plasma protein was denatured while the equilibrium of TPT structure was maintained, arid, then, the sample was centrifuged at 10000 rpm for 10 minutes so that protein components were precipitated, and, then, the concentration of lactone ring-opened TPT and that of lactone ring-closed TPT in supernatant were measured with HPLC. Results are shown in Table 2 below. An aqueous solution of TPT (pH 3; phosphate hydrochloride buffer) was used for control.

Table 2. Change with time of the ratio of ring-closed TPT in 50 % human plasma

| Item | 0 Hour | 2 Hours | 4 Hours |
|---|---|---|---|
| Control | 98.8 % | 20.8 % | 13.4 % |
| Nanoparticles | 98.1 % | 33.7 % | 25.4 % |

Comparative Example

(to examine the influence of the addition of PLA)

Polyethylene glycol (molecular weight: 12000) - co-poly(benzyl-L-aspartate) (degree of polymerization of aspartic acid: 50) having an esterification rate of 50 % (hereinafter referred to as PEG-PBLA 12-50 P.H. 50 %) was used as block copolymer. Five mg of PEG-PBLA 12-50 P.H. 50 % and 1 mg of TPT were put into a 9 mL screwed tube bottle, and were then dissolved in 1 mL of methanol. The resultant solution was then dried and solidified by nitrogen blowing, and a film-like matter was obtained. To the film-like matter, 3 mL of water was added, and the resultant mixture was stirred vigorously for a whole day and night at 4°C. The mixture was thereafter subjected to sonification for three minutes, and, thus, TPT-containing polymer micelle formulation was prepared. Furthermore, unencapsulated drug was removed by Amicon Ultra, an ultrafiltration membrane (MWCO: 100,000).

Encapsulating rate was measured with an ultrafiltration membrane (Microcon YM-100; MWCO: 100,000). One hundred μL of sample was set in Microcon YM-100, and was then centrifuged for five minutes at 4°C, 10000 rpm, to give a filtrate. The amount of TPT in the sample immediately after prepared (A) and the amount of TPT in the filtrate (B) were measured with HPLC, and, thus, drug-encapsulating rate was calculated according to the following formula. As a result, it was found that 81.2 % of topotecan had been encapsulated in particles.

$$\text{Encapsulating rate (\%)} = \frac{(A - B) \times 100}{A}$$

Particle size was measured with DLS. It was found that average particle size was about 69 nm. The stability (releasing rate) of the obtained TPT-containing nanoparticle formulation was evaluated in the following manner.

TPT-encapsulating rate in nanoparticles when PBS buffer solution had been added was measured, and, thus, the rate of releasing of TPT caused by the addition of salt was determined. To 90 μL of sample from which unencapsulated drug had been removed by ultrafiltration membrane in the above-mentioned manner, 10 μL of PBS buffer solution was added, and, then, encapsulating rate was measured in the same manner as mentioned above, and, thus, the ratio of TPT remaining in the particles was confirmed. As a result, it was found that only 4.2 % of TPT had remained in nanoparticles.

Example 3

Polyethylene glycol (molecular weight: 12000) - co-poly(benzyl-L-aspartate) (degree of polymerization of aspartic acid: 50) (hereinafter referred to as PEG-PBLA 12-50) was used as block copolymer. 50 mg of PEG-PBLA 12-50, 1 mg of TPT and 20 mg of PLA-20000 were put into a 9 mL screwed tube bottle, and were then dissolved in 1 mL of acetone. The resultant solution was then dried and solidified with nitrogen blowing, and a film-like matter was obtained. To the film-like matter, 3 mL of water was added, and the resultant mixture was stirred vigorously for a whole day and night at 4°C. The mixture was thereafter subjected to sonification for five minutes, and, then, large particles and extraneous matters were filtered out with a membrane having a pore size of 0.8 μm, and, thus, TPT-containing micellar nanoparticles were prepared. Furthermore, unencapsulated drug was removed by Amicon Ultra, an ultrafiltration membrane (MWCO: 100,000).

Encapsulating rate was measured with an ultrafiltration membrane (Microcon YM-100; MWCO: 100,000). One hundred μL of sample was set in Microcon, and was then centrifuged for five minutes at 4°C, 10000 rpm, to give a filtrate. The amount of TPT in the sample immediately after prepared (A) and the amount of TPT in the filtrate (B) were measured with HPLC, and, then, drug-encapsulating rate was calculated according to the following formula. As a result, it was found that 97.6 % of topotecan had been encapsulated in nanonparticles.

$$\text{Encapsulating rate (\%)} = \frac{(A - B) \times 100}{A}$$

Particle size was measured with DLS. It was found that average particle size was about 155 nm. The drug stability (releasing rate) of TPT-containing nanoparticles was evaluated in the following manner.

TPT-encapsulating rate in nanoparticles when PBS buffer solution had been added was measured, and, thus, the rate of inhibition of releasing of TPT caused by the addition of salt was determined. To 90 μL of sample, 10 μL of PBS buffer solution was added. Then, encapsulating rate was measured in the same manner as mentioned above, and, thus, the ratio of TPT remaining in the particles was confirmed. As a result, it was found that 88.1 % of TPT had remained in nanoparticles.

Fifty μL of TPT-containing micellar nanoparticle formulation or 50 μL of aqueous TPT solution (0.3 mg/mL) containing 50 mg/mL of PEG-4000 and 50 mg/mL of mannitol was freeze-dried, and was dissolved in 1 mL of 50 % human plasma (diluted with PBS), and were incubated at 37°C. For the purpose of investigating the ratio of ring-opening and ring-closing of TPT with lapse of time, 100 μL of incubated sample was taken out after 0 hour, 2 hours and 4 hours, and was added to 900 μL of methanol. Plasma protein was denatured while the equilibrium of TPT structure was maintained, and, then, the sample was centrifuged at 10000 rpm for 10 minutes so that protein components were precipitated, and, then, the concentration of lactone ring-opened TPT and that of lactone ring-closed TPT in supernatant were measured with HPLC. Results are shown in Table 3 below.

Table 3. Change with time of the ratio of ring-closed TPT in 50 % human plasma

| Item | 0 Hour | 2 Hours | 4 Hours |
|---|---|---|---|
| Control | 98.9 % | 18.6 % | 14.6 % |
| Nanoparticles | 100.0 % | 70.1 % | 61.2 % |

Example 4

Polyethylene glycol (molecular weight: 12000) - co-poly(octyl-L-aspartate) (degree of polymerization of aspartic acid: 25) (hereinafter referred to as PEG-PLAC8 12-25) was used. 20 mg of PEG-PLAC8 12-25, 1 mg of TRH and 20 mg of PLA-20000 were put into a 9 mL screwed tube bottle, and were then dissolved in a mixture of 1 mL of acetone and 80 μL of methanol. The resultant solution was then dried and solidified with nitrogen blowing, and a film-like matter was obtained. To the film-like matter, 3 mL of water was added, and the resultant mixture was stirred vigorously for a whole day and night at 4°C. The mixture was thereafter subjected to sonification for five minutes, and, thus, TRH-containing nanocapsules were prepared.

Encapsulating rate was measured with an ultrafiltration membrane (Microcon YM-100; MWCO: 100,000). 100 μL of sample was set in Microcon, and was then centrifuged for five minutes at 4°C, 10000 rpm, to give a filtrate. The amount of TRH in the sample (A) and the amount of TRH in the filtrate (B) were measured with HPLC, and, thus, drug-encapsulating rate was calculated according to the following formula. As a result, it was found that 35.3 % of TRH had been encapsulated in nanonparticles.

$$\text{Encapsulating rate (\%)} = \frac{(A - B) \times 100}{A}$$

The drug stability (releasing rate) of TRH-encapsulating nanoparticles was evaluated in the following manner.

TRH-encapsulating rate in nanoparticles when PBS buffer solution had been added was measured, and, thus, the rate of inhibition of releasing of TRH caused by the addition of salt was determined. To 90 μL of sample, 10 μL of PBS buffer solution was added. Immediately after stirring, encapsulating rate was measured in the same manner as mentioned above, and, then, the ratio of TRH remaining in the particles was confirmed. As a result, it was found that 22.9 % of TRH had remained in nanoparticles.

Comparative Example

(to examine the influence of the addition of PLA)

Polyethylene glycol (molecular weight: 12000) - co-poly(octyl-L-aspartate) (degree of polymerization of aspartic acid: 25) (hereinafter referred to as PEG-PLAC8 12-25) was used. 20 mg of PEG-PLAC8 12-25 and 1 mg of TRH were put into a 9 mL screwed tube bottle, and were then dissolved in a mixture of 1 mL of acetone and 80 μL of methanol. The resultant solution was then dried and solidified by nitrogen blowing. To the resultant solid matter, 3 mL of water was added, and the resultant mixture was stirred vigorously for a whole day and night at 4°C. The mixture was thereafter

subjected to sonification for five minutes, and, thus, TRH-containing nanoparticles were prepared.

Encapsulating rate was measured with an ultrafiltration membrane (Microcon YM-100). 100 μL of sample was set in Microcon YM-100, and was then centrifuged for five minutes at 4°C, 10000 rpm, to give a filtrate. The amount of TRH in the sample (A) and the amount of TRH in the filtrate (B) were measured with HPLC, and, thus, drug-encapsulating rate was calculated according to the following formula. As a result, it was found that 8.3 % of TRH had been encapsulated in particles.

$$\text{Encapsulating rate (\%)} = \frac{(A - B) \times 100}{A}$$

The stability of the obtained TRH-containing nanoparticles was evaluated in the following manner.

TRH -encapsulating rate in nanoparticles when PBS buffer solution had been added was measured, and, thus, the rate of releasing of TRH caused by the addition of salt was determined. To 90 μL of sample, 10 μL of PBS buffer solution was added, and, then, encapsulating rate was measured in the same manner as mentioned above, and, thus, the ratio of TRH remaining in the particles was confirmed. As a result, it was found that only 2.3 % of TRH had remained in the particles.

Example 5

Polyethylene glycol (molecular weight: 12000) - co-poly(octyl-L-aspartate) (degree of polymerization of aspartic acid: 25) (hereinafter referred to as PEG-PLAC8 12-25) was used. 10 mg of PEG-PLAC8 12-25, 1 mg of TPT and 10 mg of PLA-20000 were put into a 9 mL screwed tube bottle, and were then dissolved in 2 mL of dichloromethane. The resultant solution was then dried and solidified with nitrogen blowing, and a film-like matter was obtained. To the film-like matter, 3 mL of water was added, and the resultant mixture was stirred vigorously for a whole day and night at 4°C. The mixture was thereafter subjected to sonification for five minutes, and, then, large particles and extraneous matters were filtered out with a membrane having a pore size of 0.8 μm, and, thus, TPT-containing micellar nanoparticles were prepared. Furthermore, unencapsulated drug was removed by Amicon Ultra, an ultrafiltration membrane (MWCO: 100,000).

Encapsulating rate was measured with an ultrafiltration membrane (Microcon YM-100; MWCO: 100,000). 100 μL of sample was set in Microcon, and was then centrifuged for five minutes at 4°C, 10000 rpm, to give a filtrate. The amount of TPT in the sample immediately after prepared (A) and the amount of TPT in the filtrate (B) were measured with HPLC, and, thus, drug-encapsulating rate was calculated according to the following formula. As a result, it was found that 99.5 % of topotecan had been encapsulated in nanonparticles.

$$\text{Encapsulating rate (\%)} = \frac{(A - B) \times 100}{A}$$

Particle size was measured with DLS. It was found that average particle size was about 258 nm. The drug stability (releasing rate) of TPT-containing nanoparticles was evaluated in the following manner.

TPT-encapsulating rate in nanoparticles when PBS buffer solution had been added was measured, and, thus, the rate of releasing of TPT caused by the addition of salt was determined. To 90 μL of sample, 10 μL of PBS buffer solution was added. Then, encapsulating rate was measured in the same manner as mentioned above, and, thus, the ratio of TPT remaining in the particles was confirmed. As a result, it was found that 100 % of TPT had remained in nanoparticles.

Fifty μL of TPT-containing nanocapsule particle formulation or its freeze-dried product was dissolved in 1 mL of 50 % human plasma (diluted with PBS), and were incubated at 37°C. For the purpose of investigating the ratio of ring-opening and ring-closing of TPT with lapse of time, 100 μL of incubated sample was taken out after 0 hour, 2 hours and 4 hours, and was added to 900 μL of methanol. Plasma protein was denatured while the equilibrium of TPT structure was maintained, and, then, the sample was centrifuged at 10000 rpm for 10 minutes so that protein components were precipitated, and, then, the concentration of lactone ring-opened TPT and that of lactone ring-closed TPT in supernatant were measured with HPLC. Results are shown in Table below.

Table 4. Change with time of the ratio of ring-closed TPT in 50 % human plasma

| Item | 0 Hour | 2 Hours | 4 Hours |
|---|---|---|---|
| Control | 98.2 % | 14.4 % | 11.9 % |
| Nanoparticles | 97.1 % | 90.6 % | 86.6 % |

Industrial Applicability

This invention provides a drug, in particular useful form for administration of drugs. This invention is therefore usable in medical industries.

## Claims

1. A composition containing drug-encapsulated nanoparticles each of which comprises three components of a) water-soluble and basic drug, b) a biodegradable polymer having at least one carboxyl group in a molecule, and c) a block copolymer having a hydrophilic segment and a hydrophobic segment.

2. A composition according to claim 1 wherein the water-soluble and basic drug is polypeptide.

3. A composition according to claim 1 wherein the water-soluble and basic drug is a water-soluble camptothecin derivative.

4. A composition according to claim 1 wherein the biodegradable polymer having carboxyl group is poly(lactic acid) or poly(lactic-coglycolic acid).

5. A composition according to anyone of claims 1 to 4 wherein the block copolymer comprises hydrophilic segment selected from the group consisting of poly(ethyleneoxide), poly(vinylalcohol), poly(vinylpyrrolidone), poly(N,N-dimethylacrylamide) and dextran, and hydrophobic segment selected from the group consisting of poly(β-alkylaspartate), poly(β-alkylaspartate-coaspartic acid), poly(β-aralkylaspartate), poly(β-aralkylaspartate-coaspartic acid), poly(γ-alkylglutamate), poly(γ-alkylglutamate-coglutamic acid), poly(γ-aralkylglutamate), poly(β-alkylaspartamide), poly(β-alkylaspartamide-coaspartic acid), poly(β-aralkylaspartamide), poly(β-aralkylaspartamide-coaspartic acid), poly(γ-alkylglutamide), poly(γ-alkylglutamide-coglutamic acid), poly(γ-aralkylglutamide), poly(γ-aralkylglutamide-coglutamic acid), poly(lactide), poly(lactide-coglycolide), poly(ε-caprolactone), poly(δ-valerolactone) and poly(γ-butyrolactone), and is capable of forming polymer micelle in an aqueous medium.

6. A composition according to claim 5 wherein the block copolymer has formula (I) or (II) as follows:

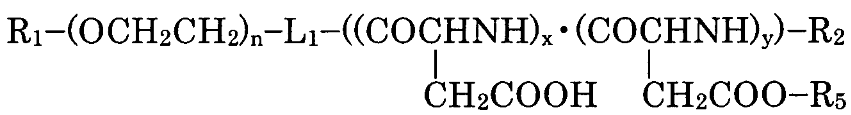

(I)

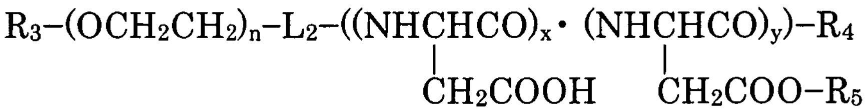

(II)

wherein $R_1$ and $R_3$ independently denote a hydrogen atom or a lower alkyl group which is either substituted by a functional group which may be protected or unsubstituted; $R_2$ denotes a hydrogen atom, a saturated or unsaturated $C_1$-$C_{29}$ aliphatic carbonyl group or an aryl carbonyl group; $R_4$ denotes a hydroxyl group, a saturated or unsaturated $C_1$-$C_{30}$ aliphatic oxy group or an aryl-lower alkyloxy group; $R_5$ denotes a benzyl group, an alkylbenzyl group or an allyl group; $L_1$ and $L_2$ independently denote a linker; n denotes an integer of 10 to 2500; and x and y are the same

or different, and each denote an integer such that the total of x and y is 10 to 300, provided that x:y falls in the range of 2-0 8-10, and that, when x is not zero, the recurring units of x are each present at random.

**7.** A composition according to claim 6 wherein $L_1$ is selected from the group consisting of -NH-, -O-, -CO-, $-CH_2-$, -O-Z-S-Z-, -O-Z-NH- and -OCO-Z-NH- (Z independently denotes a $C_1$-$C_4$ alkylene group), and $L_2$ is selected from the group consisting of -OCO-Z-CO-, -NHCO-Z-CO- and -O-Z-NH- (Z independently denotes a $C_1$-$C_4$ alkylene group).

**8.** A composition according to anyone of claims 1 to 7 wherein a mixture of the drug a) and the biodegradable polymer b) is encapsulated into nanoparticle formed by the block copolymer c).

**INTERNATIONAL SEARCH REPORT**

International application No.
PCT/JP2004/013256

A. CLASSIFICATION OF SUBJECT MATTER
Int.Cl[7] A61K9/51, 47/32, 47/34, 47/36, 47/42, A61P5/10, 5/14, 5/24, 25/04, 35/00

According to International Patent Classification (IPC) or to both national classification and IPC

B. FIELDS SEARCHED

Minimum documentation searched (classification system followed by classification symbols)
Int.Cl[7] A61K9/51, 47/32, 47/34, 47/36, 47/42, A61P5/10, 5/14, 5/24, 25/04, 35/00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)
CAPLUS(STN), REGISTRY(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 03/033592 A1 (SAMYANG CORP.), 24 April, 2003 (24.04.03), Full text; particularly, Claims 1 to 21; page 5 & CA 2463172 A | 1-5,8<br>6,7 |
| Y | US 2001/0014354 A1 (NANO CARRIER CO., LTD.), 16 August, 2001 (16.08.01), Full text; particularly, Claim 9 (Family: none) | 1-8 |
| Y | JP 2002-154963 A (Yakult Honsha Co., Ltd.), 28 May, 2002 (28.05.02), Full text; particularly, Claims 1 to 5 (Family: none) | 1-8 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>09 November, 2004 (09.11.04) | Date of mailing of the international search report<br>30 November, 2004 (30.11.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/013256

(Continuation). DOCUMENTS CONSIDERED TO BE RELEVANT

| Category* | Citation of document, with indication, where appropriate, of the relevant passages | Relevant to claim No. |
|---|---|---|
| E,A | JP 2003-342167 A (Nano Carrier Co., Ltd.), 03 December, 2003 (03.12.03), Full text; particularly, Claims 1 to 12 (Family: none) | 1-8 |

210 (continuation of second sheet) (January 2004)